Europäisches Patentamt

European Patent Office

Office européen des brevets

(19)

(11) Veröffentlichungsnummer: **0 141 150 B1**

(12) **EUROPÄISCHE PATENTSCHRIFT**

(45) Veröffentlichungstag der Patentschrift: 28.08.91

(51) Int. Cl.⁵: **H01G 7/02, A61N 1/10,** //A61L15/00

(21) Anmeldenummer: 84110603.2

(22) Anmeldetag: 06.09.84

(54) **Verfahren zur Herstellung elektrisch permanent geladenen Schichtmaterials.**

(30) Priorität: 14.09.83 DE 3333083

(43) Veröffentlichungstag der Anmeldung:
15.05.85 Patentblatt 85/20

(45) Bekanntmachung des Hinweises auf die Patenterteilung:
28.08.91 Patentblatt 91/35

(84) Benannte Vertragsstaaten:
AT BE CH DE FR GB IT LI LU NL SE

(56) Entgegenhaltungen:
EP-A- 0 060 687
DE-A- 1 901 605
DE-A- 3 120 576
FR-A- 2 374 918
US-A- 4 127 681

(73) Patentinhaber: Metev Gesellschaft für Entwicklung und Verwertung med. Bedarfs GmbH & Co. KG
Rietbergerstrasse 26 Postfach 18 05
W-4840 Rheda-Wiedenbrück(DE)

(72) Erfinder: Stüer, Willy
Christoph-Siebe-Strasse 4
W-4840 Rheda-Wiedenbrück(DE)

(74) Vertreter: Schumacher, Horst, Dr. Dipl.-Phys. et al
Patentanwälte Dipl.-Phys. Dr. Peter Palgen
Dipl.-Phys. Dr. H. Schumacher Frühlingstrasse 43A (Ecke Holunderweg)
W-4300 Essen 1(DE)

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung eines elektrisch permanent geladenen, in einem elektrischen Gleichspannungsfeld verfestigten Schichtmaterial zur Erzielung therapeutischer Wirkungen.

Elektrisch permanent geladene Materialien besitzen ein elektrisches Feld, das in verschiedensten Anwendungsgebieten zum Tragen kommen kann, etwa bei Bandagen, insbesondere für Heilbehandlungen des menschlichen Körpers oder für Tiere, aber auch als Membranen für Mikrofone und für andere Anwendungsfälle, in denen in geringem Abstand einer Körperoberfläche elektrische Felder benötigt werden.

Als elektrisch permanent geladene Materialien sind vor allem Elektrete bekannt geworden, dies sind Dielektrika mit permanenter dielektrischer Polarisation. In bestimmten Harzen und anderen polaren verfestigbaren Flüssigkeiten bleibt die in geschmolzenem, also flüssigem, Zustand durch ein starkes elektrisches Feld erzwungene Ausrichtung der molekularen Dipole bestehen, wenn nach der Erstarrung das Feld abgeschaltet wird; der sich ergebende Zustand ähnelt den Permanentmagneten, die sich durch zwei in ihren Wirkungen gegensätzliche Polflächen auszeichnen. Normalerweise gleichen sich Ladungen, die sich auf der Oberfläche eines solchen Isolierstoffes befinden, infolge einer meist vorhandenen geringen Eigenleitfähigkeit des Isolierstoffes allmählich aus. In den letzten Jahren ist es jedoch gelungen, bei dünnen Kunststoffolien durch Behandlung mit ionisierenden Strahlen, z.B. Elektronenstrahlen, die Ladungsträger zu entfernen, die die Eigenleitfähigkeit bewirken. Dabei erhält die Kunststoffolie eine durch Dipole erzeugte Oberflächenladung, die sie dauernd behält. Dieses Herstellungsverfahren ist jedoch aufwendig und läßt sich in solchen Fällen nicht durchführen, in denen die ionisierenden Strahlen andere Stoffeigenschaften zerstören könnten.

Aus der deutschen Patentschrift 19 01 605 ist es bekannt, zur Erzielung therapeutischer Wirkungen elektrisch permanent geladene Schichtmaterialien der eingangs genannten Art dadurch herzustellen, daß sich die Ausgangsstoffe in einem elektrischen Gleichspannungsfeld auf einer elektrisch leitenden, auf etwa 2.000 V aufgeladenen Platte verfestigen. Diese bekannten elektrisch permanent polarisierten Materialschichten sind relativ dick und ihr elektrisches dipolares Feld baut sich mit der Zeit ab.

Aus der deutschen Offenlegungsschrift 30 39 561 ist es bekannt, für die Herstellung von Elektreten ein Material aus Hexamethyldisiloxan zu verwenden, um daraus einen dünnen Film im Wege der Plasmapolymerisation auf einer Unterlage, z.B. einer Aluminiumfolie, abzulagern. Für diesen Prozeß des Ablagerns ist es zumeist erforderlich, die Unterlage elektrisch vorzuspannen, da bei der Plasmapolymerisation positiv und negativ geladene Ionen des Ausgangsmaterials entstehen, von denen die gewünschte Ionenart abgelagert und die nicht gewünschte Ionenart entfernt werden soll. Der so gebildete Film kann nachfolgend einer thermoelektrischen Behandlung ausgesetzt werden, in der der Film erhitzt wird, einem elektrischen Feld ausgesetzt wird, und dann gekühlt wird, während er sich unter dem Einfluß des Feldes befindet. Sowohl das Plasmapolymerisieren als auch das Niederschlagen von Ionen zu einem Film in einem elektrischen Feld, wie auch die thermoelektrische Nachbehandlung, sind aufwendige Verfahrensschritte, die sich aus wirtschaftlicher Sicht offensichtlich nur deshalb noch vertreten lassen, weil diese bekannten Dünnschichtfilme auf dem Elektronikgebiet, insbesondere auf dem Gebiet der Wandler, speziell der elektroakustischen und elektromechanischen Wandler, Anwendung finden. Im übrigen sind die Oberflächenpotentiale solcher Filme auch relativ schwach - es wurden Wert von nur bis zu 185 V festgestellt - und das Oberflächenpotential baut sich langsam ab, so daß es nach einigen Monaten nur noch mehrere Volt beträgt.

Aus der deutschen Offenlegungsschrift 21 42 633 ist ein Verfahren zum Erzeugen folienförmiger Elektrete bekannt, gemäß dem polymerisierte Kunststoffolien zwischen Elektroden bei erhöhter Temperatur einem elektrischen Gleichspannungsfeld von 70 kV ausgesetzt werden, welches weiter angelegt bleibt, während die Folie wieder abgekühlt wird. Danach wird das Elektret mit Wasser behandelt, um unstabile Komponenten einer Heteroladung und die Homoladung des Elektreten zu beseitigen und lediglich eine stabile Heteroladung aufrechtzuerhalten. Auch bei diesem bekannten Verfahren wird demnach eine thermoelektrische Behandlung einer mehr oder minder dicken, bereits fertigen Folie aus Kunststoff, bei der nach der Behandlung mit Wasser Heteroladungen von ebenfalls nur etwa 200 V - wie in der DE-OS 30 39 561 - festgestellt wurden. Die Behandlung in Wasser ist außerordentlich langwierig und dauert regelmäßig mehr als 100 Stunden.

Aus der deutschen Offenlegungsschrift 21 34 571 ist es bekannt, einen für den Elektronikbereich geeigneten Dünnfilmelektreten an einer oder beiden Oberflächen mit einer Schicht einer metallischen Legierung zu bedecken. Hierzu wird auf eine oder beiden Oberflächen eines dünnen Filmes eines hochisolierenden Ausgangsmaterials für den Elektreten eine metallische Schicht aufgebracht, insbesondere aufgedampft, und anschließend wird eine derartige Sandwichkonstruktion in einem elektrischen Gleichspannungsfeld einer thermoelektrischen Behandlung unterzogen, bei der Gleichspannungen zwischen 4,5 und 6,0 kV angewendet wurden. Mit derartigen metallbeschichteten Elektreten können dauerhafte Spannungen bis 1,2 kV realisiert werden. Auch dieses Herstellungsverfahren ist wegen des erforderlichen Metallaufdampfens sehr aufwendig und insbesondere für faserförmige Trägermaterialien, insbesondere aus Kunststoffmaterial, nicht geeignet.

Aus der deutschen Offenlegungsschrift 28 29 225 ist ein Elektret für einen elektromechanischen Wandler bekannt, der auf einer speziellen Zwischenschicht als Trägermaterial folienartig aufgetragen ist und in dieser Form für Bandagen und dgl. zur Erzielung therapeutischer Wirkungen ungeeignet ist. Die als Trägerschicht dienende Zwischenschicht weist einen hohen spezifischen elektrischen Widerstand auf und kann aus einem Elastomer, insbesondere auf der Basis von Silicon bestehen. In diesem Falle ist die Trägerschicht, also nicht der Elektret, auf Siliconbasis hergestellt.

Davon ausgehend liegt der Erfindung die neue **Aufgabe** zugrunde, ein Schichtmaterial der eingangs genannten Art mit einem schichtförmigen Trägermaterial aus Textilfasern herzustellen, dessen Herstellung einfach ist und das sein elektrisches Feld dauerhaft behält. Vor allem ist es erwünscht, daß bei einem solchen Schichtmaterial nur eine, vorgebbare, Ladungsträgerart in Form eines elektrischen Feldes zum Tragen kommt.

Als technische **Lösung** wird ein Herstellungsverfahrens der im Patentanspruch 1 genannten Art vorgeschlagen. Die Erfindung beruht also darauf ein elektrisch permanent geladenes Schichtmaterial zur Erzielung therapeutischer Wirkungen dadurch bereitzustellen, daß ein in einem Lösungsmittel gelöster polymerer Kunststoff auf einem schichtförmigen Trägermaterial aus Textilfasern als relativ dünne Schicht niedergeschlagen und anschließend in einem elektrischen Gleichspannungsfeld zur Kondensation gebracht wird.

Das erfindungsgemäße Herstellungsverfahren hat den Vorteil, daß die dünne Schicht aus polymerem Kunststoff auf dem textilen Trägermaterial ohne aufwendige ionisierende Bestrahlung ein von seiner Oberfläche ausgehendes, auch über lange Zeiträume permanent bestehenbleibendes, relativ starkes elektrisches Feld besitzt, daß fernerhin die elektrisch permanent geladene Schicht sehr dünn sein kann und auf dem textilen Trägermaterial auf einfache Weise, sehr dauerhaft und außerordentlich fest aufgebracht werden kann und in dieser Form als Bandage oder als jedes andere beliebige Textilstück verarbeitbar ist, welches an dem therapeutisch zu behandelnden Lebewesen körpernah zur Anwendung kommt.

Als polymere Kunststoffe kommen erfindungsgemäß bevorzugt Polyorganosiloxane, PTFE, Polyester, Polypropylen oder kondensierbare Siliconharze in Frage. Diese polymeren Kunststoffe sind vor dem Polymerisieren in an sich bekannten Lösungsmitteln gelöst, wobei zum Auftragen besonders feiner Schichten dieses Ausgangsmaterials auf das textile Trägermaterial so viel Lösungsmittel zugesetzt wird, daß die Lösung mittels einer an sich bekannten Spritzpistole als dünner Nebel auf das textile Trägermaterial aufgesprüht werden kann; hierdurch werden auch sehr feine Textilfasern in ausreichendem Umfang mit dem polymeren Kunststoff umgeben und das textile Trägermaterial behält seine Geschmeidigkeit in dem für die Weiterverarbeitung erforderlichen Umfang.

Die als polymerer Kunststoff erfindungsgemäß besonders geeigneten Polyorganosiloxane werden allgemein als Silicone bezeichnet, sie umfassen eine Gruppe von synthetischen polymeren Verbindungen, in denen Siliciumatome über Sauerstoffatome verknüpft und die restlichen Valenzen des Siliciums durch Kohlenwasserstoffreste (meist Methyl-, seltener Äthyl-, Propyl-, Phenylgruppen u.a.) abgesättigt sind. Demnach ist der Typus des einfachen linearpolymeren Silicons nach dem Schema $(R_2SiO)_x$ aufgebaut.

Es hat sich nun überraschenderweise gezeigt, daß in einem elektrischen Feld verfestigte Materialschichten aus Polyorganosiloxanen, aber auch aus als Teflon® im Handel bekanntem PTFE, oder aus Polyester, Polypropylen oder kondensierten Siliconharzen, nicht nur ohne aufwendige ionisierende Bestrahlung ein von ihrer Oberfläche ausgehendes, auch über lange Zeiträume und nicht nur im Vakuum permanent bestehenbleibendes, relativ starkes elektrisches Feld besitzen, sondern daß dünne, vor allem sehr dünne, Schichten aus einem solchen Material sehr einfach hergestellt werden können und auf einem schichtförmigen Trägermaterial aus Textilfasern einfach, dauerhaft und außerordentlich fest aufgebracht werden können; sie stellen insofern eine neue Möglichkeit der "Oberflächenveredlung" in dem Sinne dar, daß sie nicht nur ihre allgemein bekannten hautsympathischen, hydrophobierenden und pflegeerleichternden Eigenschaften bei Textilien entfalten, insbesondere ohne die Elastizität und Flexibilität des oberflächenbehandelten Trägerwerkstoffes zu beeinflussen, sondern sie haben auch die überraschende Eigenschaft, nach allen Seiten hin ein auf derselben Ladungsart beruhendes elektrisches Feld aufzuweisen, wobei zudem beim Herstellen des Schichtmaterials die Polarität dieses elektrischen Feldes frei gewählt werden kann.

Bei einer praktischen Ausführungsform der Erfindung beträgt die aufgetragene Schichtdicke der Schicht 0,01 bis 100 $\mu$m, die Eigenfestigkeit der Schicht kann dadurch vorgegeben werden, daß der Vernetzungsgrad des polymeren Kunststoffes in an sich bekannter Weise variiert wird. Z.B. lassen sich Polyorganosiloxane nach ihren Anwendungsgebieten in Öle, zähplastische bis dünnflüssige vernetzbare Polymere, Harze und salzartige Verbindungen einteilen. Während die Öle zumeist aus linearpolymeren Dimethylsiloxanen bestehen und gewöhnlich klare, farblose, neutrale, geruchsfreie, hydrophobe Flüssigkeiten mit einem Molekulargewicht zwischen 1.000 und 150.000 darstellen und z.B. als Siliconimprägniermittel, insbesondere für Textilien, aber auch zum Hydrophobieren von Glas (z.B. in der Pharmacie) oder von Keramik dünn auf ein Trägermaterial aufgetragen werden und dort nur zu einem geringen Grad vernetzen, also sich verfestigen, stellen die gewöhnlichen in der Technik verwendeten Siliconharze mehr oder minder vernetzte Polymethyl- oder Polymethylphenylsiloxane dar, deren Elastizität und Wärmebeständigkeit mit dem Gehalt an Phenylgruppen steigt, da reine Methylsiliconharze relativ spröde und mäßig wärmebeständig sind. Solche Siliconharze gelangen gewöhnlich in vorkondensierter Form in den Handel, sie werden generell mit Hilfe von Kondensationskatalysatoren, häufig bei erhöhter Temperatur, kondensiert. Siliconharzlösungen können besonders einfach im Sprühverfahren auf ein Trägermaterial aufgesprüht werden und sich dort zu einer Schicht verfestigen, was die Oberfläche des Trägermaterials wasserabweisend macht, ohne dessen Poren zu verstopfen und die Atmung zu behindern.

Als Ausgangsmaterial für die Polyorganosiloxangewinnung dienen Organochlorsilane. Durch Hydrolyse bilden sich Silanole, welche - besonders bei erhöhter Temperatur und in Gegenwart von Katalysatoren direkt oder nach Überführung in Cyklosiloxane - zu dem gewünschten Endprodukt kondensieren; als Ausgangsmaterial hierfür sind besonders die Methylchlorsilane von besonderer Bedeutung. - Ingesamt ergeben sich sehr vielfältige Möglichkeiten von den Ausgangsstoffen oder von mehr oder minder vernetzten, bereits vorkondensierten Produkten ausgehend, ein mehr oder minder eigenfeste Schicht, insbesondere als Folie oder als dünner, aber fester Oberflächenfilm auf einem Trägermaterial herzustellen.

Da die in dem Lösungsmittel gelösten Ausgangsstoffe für die Schicht als noch flüssiges Produkt auf das schichtförmige Trägermaterial aus Textilfasern aufgetragen werden und anschließend die Verfestigung unter dem Einfluß eines elektrischen Feldes erfolgt, wird eine sehr feste und dauerhafte Verbindung zwischen der Schicht und dem schichtförmigen Trägermaterial geschaffen. Besonders bei der Verwendung von Polyester als Trägermaterial kommen die elektrischen und mechanischen besonders vorteilhaften Eigenschaften der Schicht - vor allem der Polyorganosiloxane und Siliconharze - besonders ausgeprägt zum Tragen.

Solches Trägermaterial aus Polyester wird durch Kondensation von Terephthalsäure und Äthylenglykol und anschließende Formgebung aus der Schmelze hergestellt, z.B. werden Polyesterfasern durch Verspinnen der Schmelze und Verstrecken hergestellt.

Vor allem mit der Schicht beschichtete Gewebe aus Polyesterfasern sind dazu geeignet, zu Bandagen, die ein positives oder negatives elektrisches Feld ausstrahlen sollen, verwendet zu werden, wobei die Kombination mit z.B. aus der deutschen Patentschrift 19 01 605 bekannten permanent elektrisch orientierungspolarisierten Kunststoffen, insbesondere Schichten aus solchen Schaumkunststoffen, die für medizinische Heilbehandlungen erforderlichen, ausreichend starken und permanent erhaltenbleibenden elektrischen Felder zu gewährleisten. Dabei kann das elektrische Feld durch entgegengesetzte elektrische Ladungen auf dem erfindungsgemäßen Schichtmaterial und der damit kontaktierten Oberfläche des Schaumkunststoffes wegen der elektrischen Isoliereigenschaften der Schicht nicht abgebaut werden, sondern es wird eine dauerhafte Verstärkung des insgesamt nachweisbaren elektrischen Feldes dieser Schichtkombination bewirkt. - Die stärksten elektrischen Wirkungen werden dann erzielt, wenn das Trägermaterial, insbesondere ein Gewebe aus Polyesterfasern, von der Schicht völlig eingehüllt ist, so daß die gesamte Gewebeoberfläche bzw. Faseroberfläche von der Umgebungsatmosphäre außerhalb der Schicht getrennt ist.

Gemäß einer Weiterbildung der Erfindung wird der vor dem Polymerisieren in dem Lösungsmittel gelöste polymere Kunststoff in dem elektrischen Feld in Gegenwart eines jeweils geeigneten und an sich bekannten Katalysators zur Kondensation gebracht. Hierbei erweist sich die Verwendung an sich bekannter Silanvernetzer besonders vorteilhaft, um eine schnelle Verfestigung zu gewährleisten. Entsprechende Ausgangsgemische sind als schnell vulkanisierende und leicht zu dünnen Filmen zu verarbeitende Einkomponentenlösungen auf dem Markt erhältlich, insbesondere als Imprägniermittel auf der Basis von Polydimethylsiloxan.

Wie bereits ausgeführt ist es nun möglich, die Schicht dauerhaft und wahlweise positiv oder negativ aufzuladen, wobei die Stärke des elektrischen Feldes und die Schichtdicke die Stärke des später nachweisbaren elektrischen Feldes der Schicht bestimmen. Es ist daher im Grunde auch möglich, eine völlige Null-Aufladung zu realisieren und damit zu bewirken, daß so beschichtete Trägermaterialien kein unerwünschtes elektrisches Feld besitzen.

Die Ausgangsstoffe der Schicht werden auf das auf einer elektrisch leitenden Platte liegende schichtförmige Trägermaterial aus Textilfasern aufgetragen und kondensiert, sobald die elektrisch leitende Platte in dem gewünschten Maß positiv oder negativ elektrisch aufgeladen ist, wobei bevorzugt eine Gegenplatte entgegengesetzter elektrischer Polarität entfällt. An einer solchen Platte werden positive oder negative Gleichspannungen gegenüber dem Erdpotential von 20 bis 80, bevorzugt 40 bis 60 kV, angelegt. Je nach Auftragmenge von bevorzugt 1 bis 10 g des zu kondensierenden Ausgangsstoffes/m² der herzustellenden Schicht werden unterschiedliche Schichtdicken erzielt, wobei etwa 3 bis 5 g eines Textilimprägniermittels auf der Basis von Polydimethylsiloxan/m² eines Gewebes aus Polyesterfasern eine völlige Umhüllung des Gewebes ergibt. - Eine positiv geladene Platte führt zu einer Schicht mit einer negativen elektrischen Ladung (durch entsprechende Feldstärkemessung nachweisbar) und umgekehrt.

Weitere Einzelheiten, Merkmale und Vorteile des Gegenstandes der Erfindung ergeben sich aus der nachfolgenden Beschreibung der zugehörigen Zeichnung, in der eine bevorzugte Ausführungsform einer erfindungsgemäß ausgebildeten Bandage dargestellt worden ist. In der Zeichnung zeigen:

Fig. 1    eine angelegte Bandage in Ansicht sowie

Fig. 2    eine Bandage im Schnitt.

Eine Bandage 1 ist in erster Linie aus einer weichen, z.B. 6 mm dicken Innenschicht 2 aus einem Schaum auf Kautschukbasis und einer äußeren Schutzschicht 3 aus einem Gewebe aus Polyesterfasern aufgebaut. Zwischen der Innenschicht und der Schutzschicht kann ein dünner Film 4 eines Textilimprägniermittels auf Siliconbasis etwa derart vorgesehen sein, daß dieser Film fest auf die Schutzschicht 3 aufgesprüht ist. Eine innere Deckschicht 5 aus einem Gewebe auf Polyacrylnitrilbasis kann die Innenschicht 2 auch gegen die Körperteile schützen, an der die Bandage anliegt.

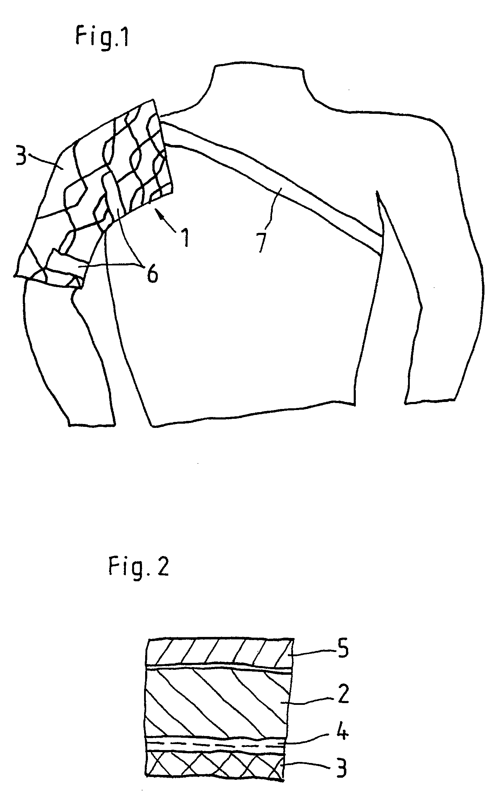

Die in Fig. 1 dargestellte Oberarmbandage ist so geschnitten, daß sie die abzudeckende Körperpartie als eine einzige geschlossene Schicht abdeckt, wobei elastische Bänder 6 mit Klettenverschlüssen an ihren freien Enden dafür sorgen, daß die Bandage hinreichend eng am Körper anlegbar ist und Bewegungen des Körpers weitgehend folgt. Es ist natürlich auch möglich, die Bandage strumpfförmig oder röhrenförmig zu gestalten und über das zu schützende Körperteil zu schieben, anstatt die Bandage als offene Fläche um die Körperpartie zu legen und die Überlappungskanten miteinander zu befestigen.

Ein Tragband 7 kann das Verrutschen der Bandage 1 verhindern. Die drei Schichten der Bandagen können miteinander vernäht sein, wobei sich ein Steppmuster in Sechseckform mit sich z.T. überlappenden Sechsecken gemäß Fig. 1 als besonders vorteilhaft erwiesen hat, um die Anpassungsfähigkeit der Bandage an verschiedenste Körperformen zu gewährleisten.

Die Innenschicht 2 wird bevorzugt in einem elektrischen Feld, z.B. gemäß der deutschen Patentschrift 19 01 605 hergestellt, insbesondere kann sie elektrisch positiv aufgeladen sein. Ebenso kann die Deckschicht 5 positiv aufgeladen oder aber elektrisch neutral sein. Demgegenüber wird die erfindungsgemäße Schutzschicht 3 aus Polyesterfasern während ihrer Herstellung innerhalb eines elektrischen Feldes von vorzugsweise 60 bis 80.000 V in Berührung mit der positiven oder negativen Feldelektrode mit dem Textilimprägnierungsmittel imprägniert. - Positive Feldelektroden bewirken eine elektrische Negativaufladung der Schutzschicht 3 und umgekehrt.

Die erfindungsgemäßen Polyesterfasern werden in üblicher Weise durch Kondensation von Terephthalsäure oder Äthylglykol und anschließendes Verspinnen der Schmelze und Verstrecken hergestellt.

Das Textilimprägnierungsmittel auf Siliconbasis ist bevorzugt eine schnell vulkanisierende Einkomponenten-Lösung, die bevorzugt mit einem Lösungsmittel aus Trichloräthylen, Benzin oder dgl. verdünnt auf die Schutzschicht 3 durch Sprühen aufgetragen wird und danach während des Trocknens vulkanisiert. Bei diesem Textilimprägnierungsmittel handelt es sich um ein im Handel unter der Bezeichnung VP 1580 erhältliches Produkt auf der Basis von Polydimethylsiloxan mit einem üblichen Katalysator sowie einem ebenfalls gebräuchlichen Silanvernetzer. In gleicher Weise haben sich weitere im Handel erhältliche Produkte auf Polyorganosiloxanbasis, wie das Silicon-Imprägniermittel VP 1581 und das Si-Finish-CT 53 L der Fa. Wacker Chemie GmbH in München, Bundesrepublik Deutschland, als Ausgangsprodukt für die Schicht erwiesen.

## Bezugszeichenliste

1    Bandage
2    Innenschicht
3    Schutzschicht
4    Film
5    Deckschicht
6    Bänder
7    Tragband

## Patentansprüche

1.    Verfahren zum Herstellen eines elektrisch permanent geladenen Schichtmaterials zur Erzielung therapeutischer Wirkungen, bei dem

a) ein in einem Lösungsmittel gelöster polymerer Kunststoff auf ein Trägermaterial aus Textilfasern aufgetragen und

b) danach in einem elektrischen Gleichspannungsfeld zur Kondensation gebracht wird.

2. Verfahren nach Anspruch 1, dadurch gekennzeichnet, daß der polymere Kunststoff ein Organosiloxan, Siliconharz, PTFE, Polyester oder Polypropylen ist.

3. Verfahren nach Anspruch 1 oder 2, gekennzeichnet durch die Verwendung eines Silanvernetzers beim Kondensieren.

4. Verfahren nach einem der Ansprüche 1 bis 3, gekennzeichnet durch die Verwendung von Polydimethylsiloxan oder die Verwendung eines Polydimethylsiloxan enthaltenden Imprägnierungsmittels als polymerer Kunststoff.

5. Verfahren nach einem der Ansprüche 1 bis 4, dadurch gekennzeichnet, daß 1 bis 10 g des zu kondensierenden, in einem Lösungsmittel gelösten, polymeren Kunststoffes/m² verwendet werden oder seine Schichtdicke 0,01 bis 100 μm beträgt.

6. Verfahren nach einem der Ansprüche 1 bis 5, dadurch gekennzeichnet, daß das Trägermaterial aus Polyester besteht.

7. Verfahren nach einem der Ansprüche 1 bis 6, dadurch gekennzeichnet, daß das zur Erzeugung des elektrische Gleichspannungsfeldes an eine Platte eine Spannung von mehr als 2kV, und, insbesondere, 20 bis 80 kV hat.

8. Verfahren nach einem der Ansprüche 1 bis 7, dadurch gekennzeichnet, daß die Lösung des polymeren Kunststoffes auf das Trägermaterial, insbesondere als feiner Nebel, aufgesprüht wird.

9. Verfahren nach einem der Ansprüche 1 bis 8, dadurch gekennzeichnet, daß die Textilfasern von dem gelösten polymeren Kunststoff eingehüllt werden.

10. Verfahren nach einem der Ansprüche 1 bis 9, dadurch gekennzeichnet, daß die Kondensation des polymeren Kunststoffes in Gegenwart eines Katalysators erfolgt.

11. Verfahren nach einem der Ansprüche 1 bis 10, dadurch gekennzeichnet, daß als Trägermaterial ein Gewebe aus Polyesterfasern verwendet wird.

12. Verfahren nach einem der Ansprüche 1 bis 11, dadurch gekennzeichnet, daß das Schichtmaterial mit einer Schicht aus Schaumkunststoff verbunden wird.

13. Verfahren nach einem der Ansprüche 1 bis 12, dadurch gekennzeichnet, daß die Kondensation auf einer elektrisch leitenden aufgeladenen Platte erfolgt.

14. Verfahren nach einem der Ansprüche 1 bis 13, dadurch gekennzeichnet, daß soviel Lösungsmittel verwendet wird, daß die Lösung mittels einer Spritzpistole aufgesprüht werden kann.

15. Verfahren nach einem der Ansprüche 1 bis 14, dadurch gekennzeichnet, daß das Lösungsmittel aus Trichloräthylen, Benzin od. dgl. besteht.

16. Verfahren nach einem der Ansprüche 1 bis 15, dadurch gekennzeichnet, daß eine schnell vulkanisierende Einkomponenten-Lösung als polymerer Kunststoff verwendet wird.

17. Verfahren nach einem der Ansprüche 1 bis 16, dadurch gekennzeichnet, daß ein bereits vorkondensierter Kunststoff als polymerer Kunststoff verwendet wird.

**Claims**

1. A process for producing a permanently electrically charged layered material for achieving therapeutic effects, in which
   a) a polymer plastic dissolved in a solvent is applied to a carrier material made of textile fibres and
   b) subsequently brought to condensation in a direct-voltage electric field.

2. A process according to Claim 1, characterised in that the polymer plastic is an organosiloxane, silicone resin, PTFE, polyester or polypropylene.

3. A process according to Claim 1 or 2, characterised by the use of a silane cross-linking agent during condensation.

4. A process according to any one of Claims 1 to 3, characterised by the use of polydimethyl siloxane - or an impregnating agent containing polydimethyl siloxane - as polymer plastic.

5. A process according to any one of the Claims 1 to 4, characterised in that 1 to 10 g of the polymer plastic/m² - which is to be condensed and is dissolved in a solvent - are used or in that its layer thickness is 0.01 to 100 μm.

6. A process according to any one of Claims 1 to 5, characterized in that the carrier material consists of polyester.

7. A process according to any one of Claims 1 to 6, characterised in that, in order to produce the direct-voltage electric field, a voltage of more than 2 kV and, in particular, 20 to 80 kV is applied to a plate.

8. A process according to any one of Claims 1 to 7, characterised in that the solution of the polymer plastic is sprayed onto the carrier material, in particular in the form of a fine mist.

9. A process according to any one of Claims 1 to 8, characterised in that the textile fibres are enveloped by the dissolved polymer plastic.

10. A process according to any one of Claims 1 to 9, characterised in that the condensation of the polymer plastic takes place in the presence of a catalyst.

11. A process according to any one of Claims 1 to 10, characterised in that a tissue of polyester fibres is used as the carrier material.

12. A process according to any one of Claims 1 to 11, characterised in that the layered material is bonded to a layer of plastic foam.

13. A process according to any one of Claims 1 to 12, characterised in that the condensation takes place on an electrically conductive charged plate.

14. A process according to any one of Claims 1 to 13, characterised in that enough solvent is used for the solution to be sprayed on by means of a spray gun.

15. A process according to any one of Claims 1 to 14, characterised in that the solvent consists of trichloroethylene, benzene or the like.

16. A process according to any one of Claims 1 to 15, characterised in that a quickly vulcanising single-component solution is used as polymer plastic.

17. A process according to any one of Claims 1 to 16, characterised in that an already pre-condensed plastic is used as polymer plastic.

**Revendications**

1. Procédé pour la fabrication d'un matériau stratifié chargé électriquement en permanence en vue d'obtenir des effets thérapeutiques, dans lequel :

    a) une matière plastique polymère dissoute dans un solvant est appliquée sur un matériau de support en fibres textiles, et

    b) est ensuite amenée à condensation dans un champ électrique de tension continue.

2. Procédé selon la revendication 1, caractérisé en ce que la matière plastique polymère est un organosiloxane, une résine de silicone, du PTFE, un polyester ou un polypropylène.

3. Procédé selon la revendication 1 ou 2, caractérisé par l'utilisation d'un agent de réticulation à base de silane pour la condensation.

4. Procédé selon l'une des revendications 1 à 3, caractérisé par l'utilisation de polydiméthylsiloxane ou l'utilisation d'un agent d'imprégnation contenant un polydiméthylsiloxane comme matière plastique polymère.

5. Procédé selon l'une des revendications 1 à 4, caractérisé en ce que l'on utilise de 1 à 10 grammes de la matière plastique polymère à condenser, dissoute dans un solvant, par mètre carré ou son épaisseur de couche est comprise entre 0,01 et 100 $\mu$m.

6. Procédé selon l'une des revendications 1 à 5, caractérisé en ce que le matériau de support est un polyester.

7. Procédé selon l'une des revendications 1 à 6, caractérisé en ce que pour produire le champ électrique de tension continue, on applique sur une plaque une tension de plus de 2 kV et notamment comprise entre 20 et 80 kV.

8. Procédé selon l'une des revendications 1 à 7, caractérisé en ce que la solution de matière plastique polymère est pulvérisée sur le matériau de support, notamment sous la forme d'un fin brouillard.

9. Procédé selon l'une des revendications 1 à 8, caractérisé en ce que les fibres textiles sont enveloppées par la matière plastique polymère dissoute.

10. Procédé selon l'une des revendications 1 à 9, caractérisé en ce que la condensation de la matière plastique polymère s'effectue en présence d'un catalyseur.

11. Procédé selon l'une des revendications 1 à 10, caractérisé en ce qu'on utilise comme matériau de support un tissu en fibres de polyester.

12. Procédé selon l'une des revendications 1 à 11, caractérisé en ce que le matériau stratifié est assemblé à une couche en mousse synthétique.

13. Procédé selon l'une des revendications 1 à 12, caractérisé en ce que la condensation s'effectue sur une plaque électriquement chargée et conductrice.

14. Procédé selon l'une des revendications 1 à 13, caractérisé en ce qu'on utilise suffisamment de solvant pour que la solution puisse être pulvérisée au moyen d'un pistolet pulvérisateur.

15. Procédé selon l'une des revendications 1 à 14, caractérisé en ce que le solvant est du trichloroéthylène, de l'essence ou un produit similaire.

16. Procédé selon l'une des revendications 1 à 15, caractérisé en ce qu'on utilise comme matière plastique polymère une solution à un composant se vulcanisant rapidement.

17. Procédé selon l'une des revendications 1 à 16, caractérisé en ce qu'une matière plastique déjà pré-condensée est utilisée comme matière plastique polymère.

Fig.1

Fig. 2